(19) **Europäisches Patentamt / European Patent Office / Office européen des brevets**

(11) **EP 4 670 622 A1**

(12) # EUROPEAN PATENT APPLICATION

(43) Date of publication:
**31.12.2025 Bulletin 2026/01**

(21) Application number: **24306034.0**

(22) Date of filing: **27.06.2024**

(51) International Patent Classification (IPC):
**A61B 5/021** $^{(2006.01)}$    **A61B 5/024** $^{(2006.01)}$
**A61B 5/02** $^{(2006.01)}$

(52) Cooperative Patent Classification (CPC):
**A61B 5/02007;** A61B 5/02133; A61B 5/024

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicants:
• **Institut National de la Santé et de la Recherche Médicale (INSERM)**
  75013 Paris (FR)
• **Université Paris Cité**
  75006 Paris (FR)
• **Assistance Publique-Hôpitaux de Paris (AP-HP)**
  75004 Paris (FR)
• **Universiteit Gent**
  9000 Gent (BE)

• **Meditronic Bakken Research Center B.V.**
  6229 GW Maastricht (NL)
• **Interuniversitair Micro-Electronica Centrum (IMEC) vzw**
  3001 Leuven (BE)

(72) Inventors:
• **BOUTOUYRIE, Pierre**
  75013 PARIS (FR)
• **BAETS, Roeland**
  9000 GENT (BE)
• **AASMUL, Soren**
  6229GW MAASTRICHT (NL)
• **DE MELIS, Mirko**
  6229GW MAASTRICHT (NL)

(74) Representative: **Maschio & Soames IP Ltd**
  **30 Carlton Crescent**
  **Southampton SO15 2EW (GB)**

(54) **APPARATUS AND METHODS FOR PROVIDING AN INDICATION OF BLOOD VESSEL PROPERTIES**

(57) Properties of a blood vessel are determined by use of a laser Doppler vibrometer in which laser beams are directed to a patient or subject's skin over two regions, the chest and a superficial artery region (for example carotid neck region) where a pulse in an underlying blood vessel may be experienced. The pulse vibrations are detected by the vibrometer and their time of arrival at the regions determined. A time difference is calculated and from an understanding of the distance of the blood vessel travelled between the two regions a propagation speed or pulse wave velocity is calculated. The speed will vary in accordance with the stiffness or elasticity of the blood vessel. Thus, the condition of the blood vessel may be determined by calculating the stiffness (or elasticity) from the propagation speed.

Fig 1

**EP 4 670 622 A1**

## Description

## Field Of The Invention

This invention relates to apparatus and methods for providing an indication of blood vessel properties. In particular, it relates to apparatus and methods for measuring arterial stiffness.

## Background Of the Invention

An early indicator of a patient's health and possible further medical condition outcomes can be the arterial stiffness. Cardiovascular diseases (CVD) are very significant in the contribution to ill health taking the lives of 17.9 million people per year amounting to over 30% of all global deaths. There are a number of factors that contribute to CVD these including arterial stiffness, arterial stenosis and desynchrony or other abnormal cardiac contraction patterns.

It has been proposed to detect arterial stiffness by measuring a subject's pulse at the carotid and femoral arteries by using pressure sensors for detecting the passage of the pulse wave (arterial tonometry or ultrasound). From a knowledge of the subject's anatomy a speed for the passage of the pulses can be determined. The faster the passage of the pulse the more rigid (or stiff) the arterial wall is and, conversely, the slower the pulse wave the more flexible (or distensible or elastic) the arterial wall is. The pressure sensors used take the form of piezoelectric elements formed into patches which may be attached to the subjects' skin by the hand of the operator, by tape or adhesive or by cuffs to press sensors against the skin at the level of large arteries. The pressure/ flow induced by the passage of the pulse deflects the piezoelectric element to provide an electrical output which is proportional to the pressure exerted by the pulse at the skin.

The femoral artery is located at groin region of the body and some patients are reticent in removing clothing to have the sensor applied to that part of the body. Further, this zone is barely accessible in some patients with obesity. This can lead to a poor patient experience and a reluctance, in some, to participate in further procedures. Further, application of the sensors to the subject's body requires cleaning protocols to prevent patient-to-patient contamination.

## Brief Summary Of the Invention

According to the invention there is provided in a first aspect apparatus for determining a property of a blood vessel comprising; a laser Doppler vibrometer configured to provide at least a first and a second laser output for direction to the chest and carotid regions of a patient, detectors for receiving at least some of the first and second laser output reflected from the region of a subject and for providing respective outputs representative thereof, a processor coupled to the detectors which processor being configured to determine from the respective outputs when a pulse wave has passed through the regions along the blood vessel and for providing a time difference of arrival of the detected pulse for the regions.

The time taken for travel of the pulse wave along the blood vessel between the regions will be dependent on properties of the blood vessel which include the elasticity or stiffness of the blood vessel. This can be used to gain an understanding of the health of the blood vessel. For example, arterial stiffness is a precursor for hypertension, and strongly conditions the future occurrence cardiovascular events. Preferably the apparatus is configured to utilise a known or estimated length of the blood vessel to further calculate the speed of the pulse wave through the vessel. Most preferably, the apparatus may be configured to calculate a value representing the stiffness or elasticity of the blood vessel.

In prior art arrangements, the carotid to femoral path over which measurements are taken includes the descending aorta, however, the ascending aorta, the most elastic vessel in the body, is excluded by this measure. Advantageously, measuring the time delay between the heart and the carotid vibrations covers the ascending aorta segment.

The time of arrival of the pulse may be determined by reference to a clock timing signal provided from one unit to another or from separate internal timing sources based on an external time source such as one provided by a GPS signal.

A display is preferably provided for displaying the determined properties to a user. In a preferred form the screen is a touch screen to allow user input.

The laser beams are preferably provided as narrow beams of limited extent in order to target a specific region beams These may be provided by a lens arrangement to image the laser source onto the skin or focus a wider beam into a narrower beam, for example in one preferred embodiment a photonics integrated circuit produces a set of divergent beams which have a diameter of about 10 micro metres with a separation at their origin of 500 micro metres. An optical system then focuses these onto a patient to image them with a separation of 5 m.m.

Advantageously, a plurality of beams are provided at each region being detected. The laser beams may be any wavelength and power levels which are safe to be directed to the skin, and safe for the retina. Visible wavelengths may be preferred in certain embodiments, since visible wavelengths will provide an indication to the user that the beams are directed at the correct regions. Many types of other wavelengths may be used including infra-red wavelengths. A preferred wavelength is 1300nm or thereabouts as this permits direct measurement on skin of different colours. Wavelengths of around 1300nm are further preferred since they advantageously increase the amount of backscattered laser light from the skin of the patient which makes detection easier than other wavelengths.

Where non-visible wavelengths are used, an aiming beam of visible light may be provided in addition. This may be formed as a pattern such as crossed beams, cross-hairs or other shapes. The pattern may be configured to provide a visible clue to the user that the distance to the skin from the sensor is within a certain distance parameter. For example, the pattern may be most readily apparent at a particular distance where it is optimally focused. For example, a converging beam may be provided such that the separation of parts of the pattern on the skin will vary with the distance to the skin. For example, two spots may converge into one at the required standoff distance.

The aiming beam may provide a shape or pattern which imparts to the user an indication of the alignment of the beams with the regions being detected. For example, where four beams are provided the images may be arranged at the corners of an imaginary square. Holographic images or other alignment patterns may be used for example a series of concentric circles may be projected.

Preferably, the beams lie on a straight line, which is projected onto the skin across the targeted artery to make up for the uncertainty of the exact position of the artery.

Alternatively, or in addition, as in the described embodiment, an aiming aid may be provided in the form of a physical stand-off which may contact the skin at the region to be detected.

In order to maintain alignment, and or, to reduce user induced vibrations, positioning means may be provided. In the described embodiments limbs are provided extending from the housings of the apparatus. These are placed into contact with the subject's skin at the region and have been found to greatly reduce induced user induced vibrations permitting more sensitive determination of the pulse wave to be made. The limbs may take the form of frames or feet or other forms. In the described apparatus the feet are permanently deployed to create a stand-off distance from the skin. However, it is envisaged that the limbs may be made deployable from stowed positions by, for example, making them telescopic or foldable. This will make apparatus more compact for storage and the limbs less vulnerable to damage.

In the preferred form, the apparatus is configured to include a unit per laser output which is handheld. In a described embodiment, the units provided means to allow the units to be fixed together by the user in a removable manner. This allows the units to be moved en bloc to detect over adjacent regions or separated to allow detection over more separated regions. Many means may be used to couple the units together, for example touch and loop fasteners, friction fittings, magnetic coupling or in the case of the described embodiment a sliding dovetail engagement feature.

The units may be linked to allow communication of data, instructions, clock signals and power therebetween. The links can include hard wire or optical fibre links, wireless communication links or a combination of these. A preferred form for a wireless communication link is a short-range wireless link such as that operating in accordance with the WiFi or Bluetooth™ protocol.

The detected vibrations at the regions of the subject can be used to provide, in addition to time of arrival, further information. The vibrations provide what may be termed a detected pulse wave which can be compared to stored pulse wave characteristics to identify anomalies which may indicate health problems such as cardiac desynchrony. The detected pulse wave may be displayed for scrutiny by the user.

The display of the apparatus may be provided on at least one of the units or on both or provided as part of further attached or coupled unit such as a microcomputer or a smart phone.

The length of the blood vessel may be provided to the apparatus by the user measuring the distance or determining the distance from tables of physiological relationships. The measure may be entered by the user at a use- interface devices such as a keyboard or by voice recognition. Where the units are coupled or integrated together the separation of the beams will be known from the physical dimension of the units and held as a predetermined value in memory. In the preferred version of the invention, the distance between the two measuring units will be measured automatically.

In a second aspect the invention provides a method for determining the pulse wave velocity in a blood vessel of a subject which method comprising the steps of; providing a laser Doppler vibrometer; directing laser light from the laser Doppler vibrometer to the chest and carotid regions of a subject; determining from received laser light scattered from the at least two regions a time of pulse wave arrival at the two or more regions; determining from the time of arrival of the pulses a time difference; and determining from the time difference and a distance between the two or more regions a propagation speed of the pulse wave in the blood vessel.

Preferably the method further comprises the step of determining from the propagation speed an elasticity or stiffness property of the blood vessel.

The regions are selected to be the chest and the neck of the subject where a carotid artery is located, this segment encompasses the ascending aorta, a vessel of interest, which is excluded by the carotid to femoral segment.

In a yet further aspect of the invention there is provided a computer readable medium comprising a set of processor implementable instructions to a carry out a method of the second aspect.

Instead of a carotid region a region having a superficial artery may be utilised.

## Brief Description Of The Drawings

Specific embodiments of the invention will now be described, by way of example only, with reference to, and as illustrated by, the accompanying drawings in which:

Figure 1 shows a first embodiment of the invention used to determine properties of a blood vessel in a human subject including a laser Doppler vibrometer;

Figure 2 shows in schematic form the laser and detector of the laser Doppler vibrometer used in the apparatus of figure 1;

Figure 3 is a further block diagram of a processor function used in the apparatus of figure 1 being used to determine a pulse wave time of arrival showing an example of a pulse wave;

Figure 4 is an explanatory figure showing the steps involved in the use of the apparatus;

Figure 5 shows a further embodiment of the invention in which two laser and detector modules are provided in one handheld unit with a fixed relationship for the separation of the regions being detected;

Figure 6 shows a yet further embodiment in which units may be coupled together or separated by the user to detect for pulse waves over relatively close together regions or more widely separated regions as the user desires;

Figure 7 shows a further embodiment of the invention in the form of a medical robotic system; and

Figures 8 and 9 show an implementation of aspects of the invention on a photonic integrated circuit

## Detailed Description Of Preferred Embodiments

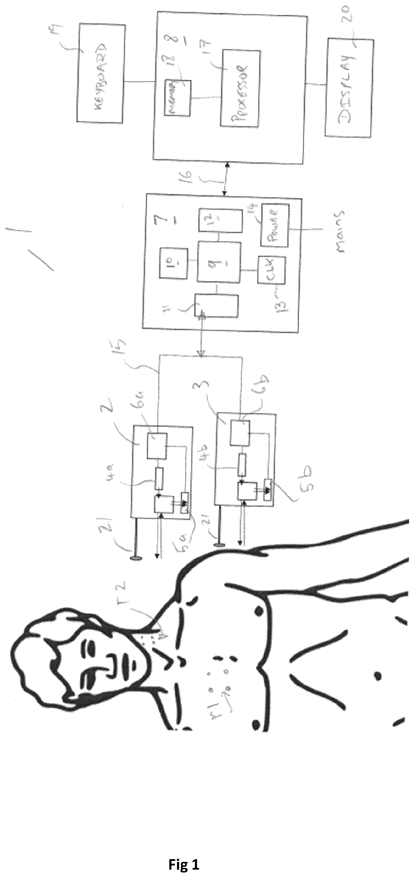

As is shown in figure 1, apparatus 1 for determining a pulse wave velocity in a patient's or subject's blood vessel comprises a pair of handheld units 2 and 3. The handheld units 2, 3 are nominally identical in function, although only one comprises the controls. Each includes a laser 4a, 4b, a detector 5a, 5b and interferometer beam splitting arrangement and a local controller 6a, 6b. The handheld units 2, 3 are operably coupled to a base unit 7 which in turn provides an output representative of a detected pulse wave velocity to microcomputer 8. The units are used to detect the arrival of a pulse wave over a chest region r1 and a region of the neck r2a (preferred), or r2b through which the carotid artery extends. Alternatively, Units can be used on any superficial artery, either coupled or separated. This includes for instance (and not restricted to) the carotid, femoral, brachial, radial, pedal, tibial posterior pulses, in any combinations.

Broadly, each of the handheld units 2, 3 detect vibrations of the targeted skin region over time from which the cardiac phenomena can be determined. The handheld unit 2 targeting the chest region detects the heart action (aortic valve closure or contraction of the ventricles). The handheld unit targeting the carotid region detects the vibration of the carotid artery and hence the arrival of the carotid wave.

The data from the handheld units is recorded with respect to time to provide sets of time series data. Form this is derived by the base unit 7 the time difference between the heart action detected at the first handheld unit monitoring the chest region and the time of arrival of the pulse wave arriving at the carotid region. The pulse wave velocity (PWV) is then calculated from the travelled length and the time difference between the heart action and the receipt of the pulse wave at the carotid region. This is more fully described below.

The base unit 7 includes a processor 9 and associated memory 10. The memory 10 may be in the form of solid state or hard drive memory. It may be ROM (Read Only Memory) or RAM (Random Access Memory) or a combination of both. The memory 10 carries a set of computer implementable instructions in machine code which when read and implemented by the processor 9 carry out the method of operation of the apparatus.

The base unit 7 further includes input and output devices 11, 12 and a clock 13. A power supply 14 is provided which may be battery or a mains power connection converted to a power level suitable for use by the base unit 7 and also the handheld units 2,3. The base unit 7 and the handheld units 2,3 are coupled together by a cord 15 which carries the power to the handheld units, and also incorporates a data bus for coupling data and instructions between the units. In alternative embodiments, a wireless communication link and protocol may be used.

Output device 12 provides the output of the processor 9 to the microcomputer by a communication link 16. The microcomputer 8 includes a microprocessor 17 with associated memory 18 and input devices 19 and a display 20. The microprocessor 15 operates in accordance with a set of machine code instructions held in memory 16. It processes the input pulse wave velocity provided from the base unit 7 using an algorithm to derive a value for the blood vessel stiffness, relative to age, blood pressure and other CV risk factors. Results will be displayed as percentiles of normal, for age, sex, and CV risk factors.

The derived stiffness value is then displayed to the user on the display 20. The input device (such as keyboard) 19 allows the user to enter data and instructions to the apparatus. In particular, the user may input the path length of the blood vessel involved between the regions of the skin r1, r2 being monitored by the handheld units 2, 3. The length may be automatically measured between units or determined by the user employing a tape measure to measure between the two regions r1, r2 on the patient's body being scanned or by other means such as the use of a patient's height and or other physiological data.

The clock 13 of the base unit 7 provides a timing signal to all units. It allows the time of arrival of pulses at each of the regions of the patient to be determined. The clock itself may be synchronised to a GPS clock signal or other timing signals.

The handheld units 2, 3 are nominally identical in terms of function (only one carries controls) so only one will be described in greater detail as such explanation will be sufficient to understand the detail of the other. The handheld unit 2 is provided with a pistol grip to facilitate handling of the unit to position it next to a region of the patient where a pulse is to be detected. The pistol grip includes a start button to provide a user input when the unit is to be placed into an operable state. In essence actuation of the start button results in a laser beam being directed to the region being monitored and for a reading to be taken by the detector. The laser 4 used is a low wattage output laser producing an infrared wavelength light of 1310nm. The laser is configured to provide four spot beams. This may be done by an optical splitter or by rapidly moving the direction of the beam to provide the spot pattern.

In the preferred form of handheld unit, a standoff leg 21 is provided. This may be a single leg or multiple legs or a frame which may be placed onto to the skin of the subject, and which assists in maintaining the handheld unit steady to permit more accurate readings. The detector 5a, is a laser detector which provides an output dependent on path length to the patient relative to a reference path length. This difference will be due to the movement of the patient's tissue at the detected region which in turn will vary as the blood vessel is disturbed by the passage of a pulse wave. Thus, by reference to the clock signal provided by clock 13 a time of arrival of the pulse may be detected at each handheld unit.

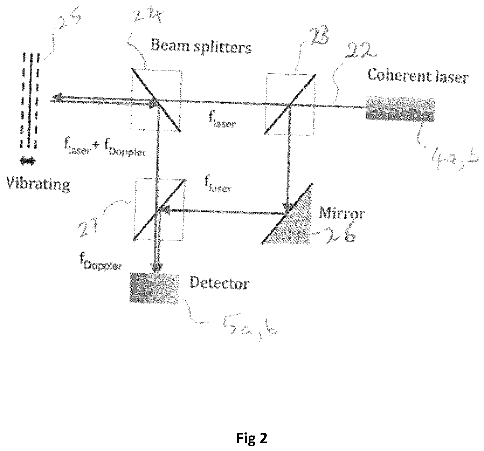

The laser and detector provide a laser Doppler interferometer or vibrometer which is shown in schematic form in figure 2. The laser provides a coherent light source. The laser beam 22 is directed to a beam splitter 23 which allows a first beam to be passed through a second beam splitter 24 to the subject's skin 25. The other part of the beam forms a reference beam which is reflected to a mirror 26 and via another beam splitter 27 to the detector 5a, b.

The interrogation beam incident on the patient's skin is scattered by the surface and part is reflected back to the beam splitter 24 where it is directed downwards (in the figure) through the beam splitter 27 to the detector 5a, b. The reflected interrogation beam and the reference laser beams cause constructive and destructive inference depending on the optical path length difference between the interrogation beam and the reference beam. Thus, the output of the detector 5a, 5b varies according to the position/vibration at the skin of the subject caused by the passage of the pulse wave. The processor 6a, b at the handheld unit interprets the signal from the detector. A signal matching process is carried out such that when the vibrations follow the characteristics of a pulse wave then the time of arrival of the pulse wave is determined. The characteristic could be an amplitude threshold being crossed or a more complex chain of amplitude variations or amplitude envelope.

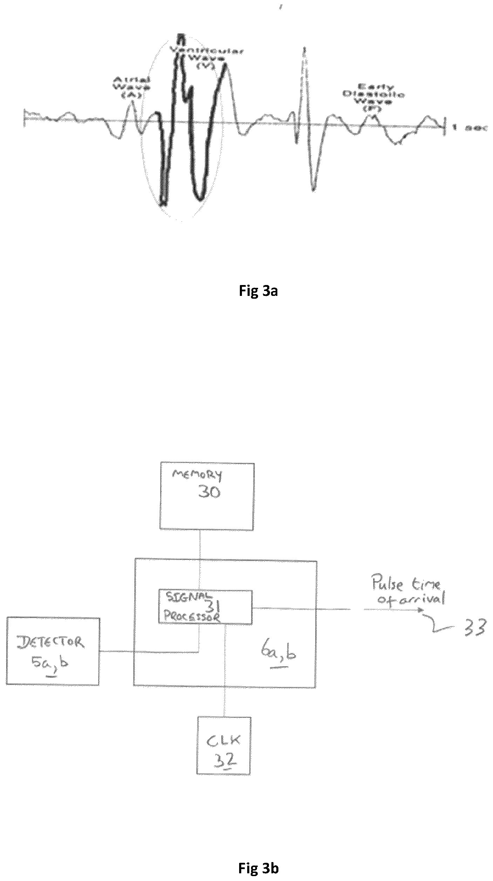

Figure 3a shows the form of a typical pulse wave (acceleration). Figure 3b shows the configuration in block diagram form of the processor 6a, 6b of the handheld unit 2, 3. A digitised version of typical pulse waves are held in memory 30, this is accessed by a signal processor 31 provided by the processor 6a, 6b. The signal processor 31 monitors the detector output and compares the output with pulse characteristics held in memory 30 or by use of algorithms to recognise features of the pulse waves these may be referred to as fiducial markers When an output matching a pulse characteristic occurs then by reference to the local clock 32 a pulse time of arrival is generated and passed to output 33. This is passed to the base unit 7 The clock 32 receives a clock reference signal from the base unit and thus in effect the time at both handheld units are synchronised. With both handheld units passing the time of arrival to the base unit 7 a time difference between the times is determine by the processor 9 of the base unit 7 and output to the microcomputer 8. From a value of the distance between the two regions r1, r2 a speed of travel of the wave can be determined and from known relationships of elasticity of vessel with speed of propagation the stiffness of the blood vessel is determined and displayed on display 20 to the user, according to the Moens Korteweg equation

$$PWV = \sqrt{\frac{E_{\text{inc}} \cdot h}{2r\rho}}$$

, further simplified by Bramwell et Hill equation $PWV = \left(\frac{\rho.dA}{dP.A}\right)^{-0.5}$ where A is arterial cross-section, dP is pulse pressure, dA/dP. A is distensibility, $\rho$ density of blood, Einc is Youngs elastic modulus, r is the radius of the artery and h is thickness of the artery wall.

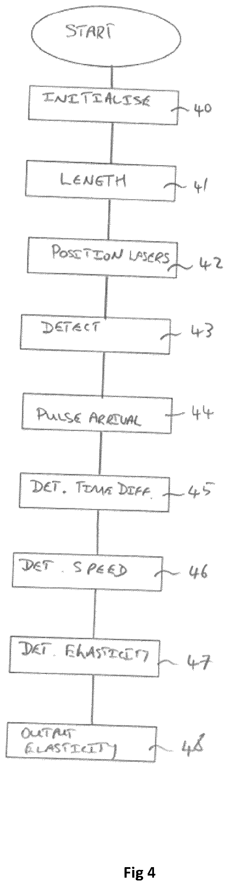

Figure 4 provides diagram of the steps performed in accordance with the invention. The steps are embodied as a computer program held within a data carrier memory associated with the processors. It should be noted that the processors may be distributed as in the described embodiment, or one processor may control all operations. The program itself may also be held in one memory or distributed across a number of memories or downloadable from remote memory over a communication link. The steps carried out by the user may result from associated prompts or instructions displayed on the apparatus for example at display 20

In a first step 40, the apparatus is provided, and the units are activated or initialised by user activation of the switches or other input devices.

In a next step 41, the user is prompted to enter a measured length for the blood vessel (if not automatically retrieved from the units. This may be provided by measuring the subject with a tape measure, or a laser distance measuring device or other means. The measurement may be taken by a tape measure from the first region r1 to the second region r2, for example. In a sense this is a direct measurement although the actual vessel length is not measured but inferred to be approximately the same as that length. An indirect measurement may be taken by for example using a subject's height measurement and chest size or other body parameters and determining from a table of known typical values an appropriate length of the blood vessel.

In step 42, the handheld units are positioned over the desired regions r1, r2 of the subject. For example, a first unit is placed over the subject's heart and a second unit is paced over the neck carotid artery. In positioning the handheld units, the standoff legs 21 are brought into contact with the skin of the subject which serves to steady the units. It will be appreciated that the orientation of the handheld units may be adjusted to position the laser beams across the targeted artery which in some embodiments may be provided by the addition of a visible light laser. Whilst the units in the described embodiments are described as handheld they may be configured to be held by tripods or other supporting structures and the invention is not limited to units which are a size, weight or configuration required for use by hand. Similarly, units can be used on any meaningful arterial segment, either in connection or not.

In step 43, the detection regions covered by the handheld units are monitored for the arrival of a pulse wave. In this the laser beam illuminates the points of the subject's skin and the backscattered light monitored as described above for the arrival of a disturbance or vibration which matches the characteristics of a pulse wave as described above.

When a pulse wave has been detected then step 44 is performed in which the time of arrival of that pulse wave at the region is determined as described above. The time difference of the time of arrival at the two regions is determined in step 45.

In step 46, the path length of the vessel and the time difference for the arrival of the pulse is used to determine the pulse wave speed or velocity. From this, in step 47, an elasticity or stiffness value of the vessel is determined, and the value is output to the user in step 48.

The vessel elasticity value or in specific embodiments the arterial stiffness may then be used by the user to provide an indication of the "health "of the blood vessel which may be used to provide treatment to the subject.

In the above-described embodiments, the vessel elasticity, arterial stiffness or other quality is determined over a length of the subject which is determined by the position of the handheld units. The distance involved will vary according to where the handheld units are placed.

Subsequent steps may be carried out to perform further calculations based on the determined elasticity or to perform diagnosis steps. It should be noted that in the above description the steps of the method may be reordered as desired, and the invention is not limited to the specific order described.

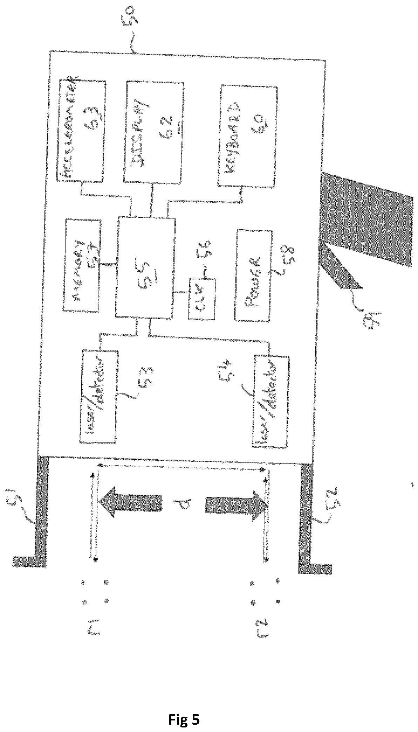

In an alternative embodiment shown in figure 5, both units are incorporated together to provide a fixed relationship and hence a fixed distance of separation for the detection zones or regions. As is shown in figure 5, one handheld unit 50 is provided with two stand-off legs 51, 52 maintaining the position form the subject's skin. Two laser and detector arrangements shown collectively as blocks 53 and 54 their structure of detector, laser and beam splitters/ reflector elements is as before described. The lasers produce the four spot patterns at two regions r1 and r2 which are separated by a distance "d" dependent upon the separation of the laser/ detector arrangements in the unit. Thus, it will be understood that this distance is fixed and hence there will be no necessity for the user to enter a path length for the vessel as such may usefully be held as a predetermined value in the memory.

The laser/detector blocks 53 and 54 provide a pulse detection signal to a processor 55 which uses a clock signal form the clock 56 to determine the respective times of arrival of the pulse wave at the two regions r1, r2 of the subject's body. The processor controls all the units' functions using a set of instructions in a form of a program held in memory 57. Power is provided by a battery 58 with the unit being activated by a trigger switch 59. The user can enter commands and data into the unit via a keyboard 60 and data is displayed on a display 62. The processor from the difference in time and the fixed distance "d" held in memory determines the velocity of the pulse wave along that part of the blood vessel. A value for the vessel elasticity or stiffness is determined and output to the user via the display 62.

The apparatus of figure 5 further provides an accelerometer block 63. This is a circuit which uses a combination of sensors such as, for example hall effect sensors, to determine movement of the apparatus. This can be utilised by the apparatus to determine if handshake or other vibration is beyond a threshold above which an accurate reading can be determined and can provide an audible or visual warning to the user via the display. Or it may be used by the processor to filter out unwanted vibrations which in effect provide "noise" to the pulse wave signal being detected. This will improve the sensitivity of the apparatus.

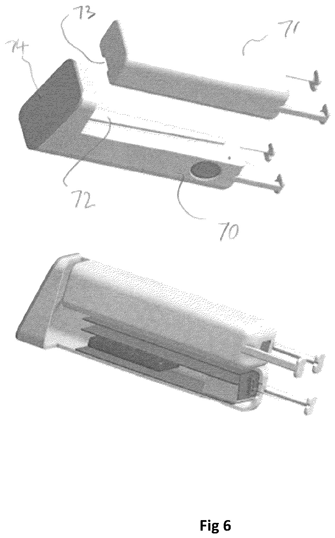

Figure 6 shows a further embodiment of the invention in which the first and the second units may be mounted together to be used in a similar manner to the embodiment of figure 5 or separated to allow detection at regions of a greater separation. The units 71, 72 are provided with male 72 and female 73 engagement features to allow the units to be removably coupled together by sliding the tapering male member 72 into the tapering slot 73. The units include communicators operating in accordance with the Bluetooth ™ communication protocol to allow commands and data to pass between the units each unit being provided with its own power source which in this case is a battery. One unit is the primary unit including the processing function to determine the time difference of arrival, speed and to calculate a value for vessel elasticity. This is displayed to the user on a display 74. The other unit 71 is a secondary unit which simply determines a time of arrival of the pulse wave and transmits this to the primary unit where it is used in the calculation as before.

The laser and detectors may provide sufficient data giving a pulse wave resolution from which abnormalities of the heart may be determined for example desynchrony. Thus, for example, taking the embodiment of figure 5, the laser detector blocks may provide not only a time of arrival of the pulse wave but an output representative of the wave characteristics itself. This may be demodulated to provide displacement data from which conditions of the heart may be determined. This is converted into a digital format and the wave passed to the processor for analysis. Both or one of the laser detector blocks 53, 54 may provide the digitised pulse wave for this analysis. The processor 55 then compares the detected wave with ideal pulse waves held in memory 57. Detected abnormalities may then be output to the display to be scrutinised by the user. Thus, the apparatus may be used to provide an indication of blood vessel condition and or heart abnormalities. Accordingly, the invention also provides in its broadest aspect apparatus and method to detect abnormal heart function such as heart desynchrony using laser detector arrangements.

In the described embodiments, two lasers are provided. However, in other embodiments one laser may be used to provide two or more laser beams to be directed to the surface and the respective reference beams. In the described embodiments, a laser is shown provided at both the handheld units. In alternative embodiments, it will be possible to couple laser light from one unit to the other by means of an optical fibre.

In the above-described embodiments, the condition of a blood vessel in an animal or human body is determined. The same apparatus may be used to determine the condition of an artificial or grafted blood vessel in an animal or human body or a model body for testing purposes prior to the blood vessel being transplanted into a patient.

In the described embodiments, the lasers are directed to regions of the patient and the laser light is scattered by the patient's skin. However, the reflectivity of the skin may be enhanced for specific purposes by application of a coating or tape.

In the above-described embodiments, the apparatus is provided as handheld or hand portable units. It will be appreciated however that features which facilitate handheld such as pistol grips and the like may be dispensed with in other embodiments still falling within the scope of the invention. The units, whilst preferably adapted to be handheld, may be made larger and heavier than could be comfortably held by hand and mounted or carried by other means including robotic or machine means. Many variations and alternatives may thus be envisioned whilst falling within the spirit and the scope of invention as described herein.

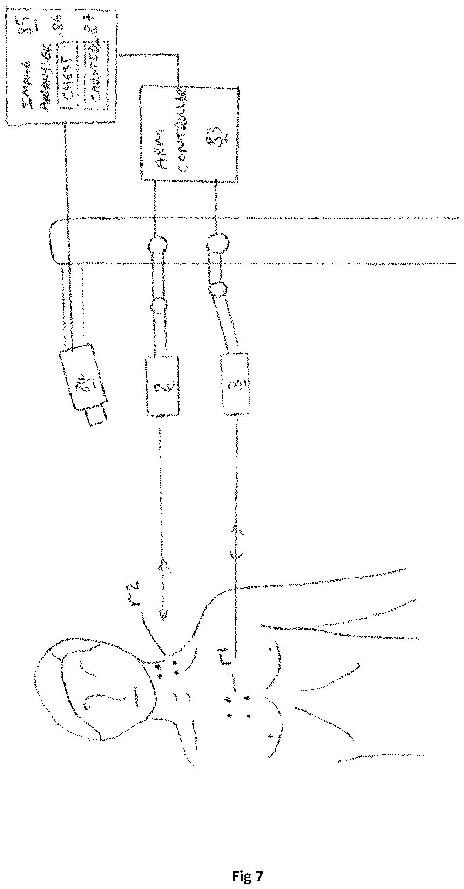

A further embodiment of the invention is shown in figure 7. In this a robotic medical system 80 is provided which includes the earlier described apparatus mounted onto robotic arms 81 and 82. The arms include a series of joints and actuators to which are controlled by an arm controller 83. At distal ends of the arms the units 2 and 3 are located. By movement of the arms, the beams may be directed to various regions of the patient "P". A camera 84 is provided . This provides an image of the patient P to a microprocessor based image analyser 85. The image analyser 84 is programmed to perform an image analysis based on machine implementable executable instructions held in local memory. The image analyser 85 comprises a block of functionality to determine from the image a chest region r1 of the patient P being a means for determining a chest region 86. A second block of functionality 87, being a means to determine a carotid region r2 of the patient determines form the image the carotid region position. The results of the determination are passed to the arm controller 83. This determines the direction or directions in which the arms need to be driven in order that the beams are directed to the chest and carotid regions. It will be appreciated that in some embodiments there will be a feedback mechanism to monitor the position of the beams on the patient in the image to further enhance the correct location of the beams. The pulse wave will be determined using the apparatus as before.

In alternative embodiments, instead of using robotic arms the beams may be directed by moving mirror arrangements. Broadly, there will be many means that may be envisioned by the skilled person for directing the beams to the chest and carotid region of the patient.

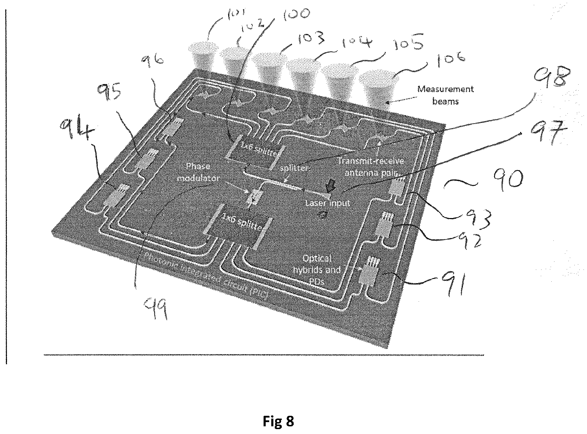

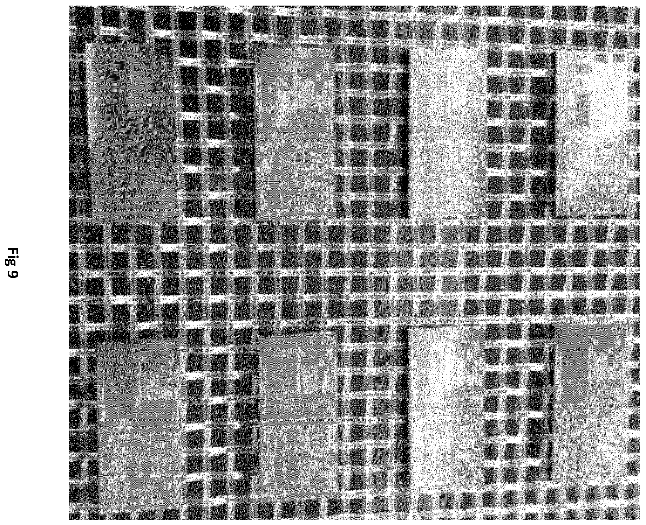

In all embodiments the detectors and beam splitting and combining components of the units as well as the light coupling antennas may be implemented in the form of a photonic integrated circuit (PIC) in which optical signals are transported via optical waveguides. For example, the units can be silicon photonic integrated circuits. These are integrated devices, manufactured by means of wafer-scale fabrication typically in a CMOS-fab, which include beam splitters and combiners, photodiodes, as well as some optical processing. The laser light source (typically a semiconductor laser diode) may also be integrated by hybrid or heterogeneous methods. A schematic figure of such a photonic integrated circuit is shown in fig. 8 and a picture of fabricated chips is shown in fig. 9. These integrated circuits are robust and reliable devices which will reduce the costs of the apparatus. In further embodiments of the invention, gyroscopes and or gimbal arrangements may be used to stabilise the apparatus.

As shown in figure 8, the photonic integrated circuit 90 comprises six optical hybrid and photodetector circuits 91 to 96. A laser input 97 allows laser generated light to be directed via a splitter 98 to a phase modulator 99. The output of the phase modulator is passed to a 1x6 splitter 100 and hence to the photodetectors 91 to 96. The splitter 97 also directs laser light to a further 1x6 splitter 100 to generate six measurement beam 101 to 106 via transmit receive antennas. These beams are coupled to an optical system for focussing at the chest and carotid regions as earlier described. The transmit receive antennas are also coupled to respective photodetectors 91 to 96.

**Claims**

1. Apparatus for determining a property of a blood vessel comprising:
   a laser Doppler vibrometer configured to provide at least a first and a second laser output;

   beam direction means for directing the laser outputs to a chest and a superficial artery region of a subject within the blood vessel is located;
   detectors for receiving at least some of the first and second laser output reflected from the region of a subject and for providing respective outputs representative thereof;
   a processor coupled to the detectors which processor being configured to determine from the respective outputs a time for the passage of a pulse wave from the chest to the superficial artery region.

2. Apparatus as claimed in claim 1 wherein the processor is further configured to determine a pulse wave velocity through and between blood vessels.

3. Apparatus as claimed in claim 2 wherein the processor is further configured to determine the elasticity of the blood vessel based on the determined speed of the pulse wave.

4. Apparatus as claimed in claim 1, 2 or 3 wherein the laser outputs provide a plurality of beams.

5. Apparatus as claimed in any one of claims 1 to 4 comprising positioning means to maintain the position of the first and second laser outputs relative to the subject.

6. Apparatus as claimed in claim 5 wherein the positioning means comprises at least one limb configured to contact the skin of the subject.

7. Apparatus as claimed in claim 6 wherein the limb depends from a housing of at least part of the laser doppler vibrometer.

8. Apparatus as claimed in any preceding claim wherein the laser doppler vibrometer is provided in at least two units with each unit providing a respective laser output and being independently positional to direct the respective laser output to the respective regions of the subject.

9. Apparatus as claimed in claim 8 wherein the units are configured to be user removably coupled such that the units may in a first mode be positioned together to detect a pulse wave over adjacent regions of the subject and in a second mode when decoupled may be positioned to detect the pulse wave at regions of the subject with a greater separation.

10. Apparatus as claimed in claim 9 wherein the units are provided with magnets which attractively couple the units together.

11. Apparatus as claimed in claim 9 or 10 wherein the units comprise male and female engagement features to enable the user to removably couple the units together.

12. Apparatus as claimed in claim 11 wherein the male and female engagement members are configured to provide a sliding dovetail joint between the units.

13. Apparatus as claimed in any preceding claim wherein the apparatus includes a processor to determine from the output of at least one of the detectors characteristics of the pulse wave and to determine therefrom abnormalities in the pulse wave.

14. Apparatus as claimed in any preceding claim further comprising: an imager for producing an image of the patient; an image analyser for determining from the image the superficial artery region and chest regions of the patient; and wherein the beam direction means is responsive to the output of the image analyser to direct the beams to the superficial artery region and chest region of the patient.

15. Apparatus as claimed in any preceding claim wherein the laser Doppler vibrometer is a silicon photonic integrated circuit.

16. Apparatus as claimed in any preceding claim wherein the superficial artery region is a carotid region.

17. A method for determining the pulse wave velocity in a blood vessel of a subject which method comprising the steps of:

   providing a laser Doppler vibrometer;

directing laser light from the laser Doppler vibrometer to two or more regions of a subject; determining from received laser light scattered from the at least the chest and superficial artery region a pulse wave velocity.

18. A method as claimed in claim 17 further comprising the step of: determining from the pulse wave velocity an elasticity or stiffness property of the artery.

19. A method as claimed in claim 16 or 17 wherein in a preliminary step: the chest and superficial artery region of the patient is identified prior to directing the laser light to those regions.

20. The method of claim 19 comprising the preliminary step of providing an image of the patient and deriving from the image the position of the chest and superficial artery region.

21. The method of any preceding claim wherein the superficial artery region is a carotid region

22. A computer medium comprising a set of processor implementable instructions to a carry out a method as claimed in any one of claims 17 to 21.

Fig 1

**Fig 2**

Atrial
Wave
(A)

Ventricular
Wave
(V)

Early
Diastolic
Wave
(P)

1 sec

**Fig 3a**

MEMORY
30

SIGNAL
31
PROCESSOR

DETECTOR
5a,b

6a,b

Pulse time
of arrival

33

CLK
32

**Fig 3b**

**Fig 4**

Fig 5

Fig 6

Fig 7

Fig 8

Fig 9

Europäisches
Patentamt

European
Patent Office

Office européen
des brevets

# EUROPEAN SEARCH REPORT

**Application Number**

EP 24 30 6034

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | LI YANLU ET AL: "Silicon photonics-based laser Doppler vibrometer array for carotid-femoral pulse wave velocity (PWV) measurement", BIOMEDICAL OPTICS EXPRESS, 22 June 2020 (2020-06-22), page 3913, XP055963855, United States ISSN: 2156-7085, DOI: 10.1364/BOE.394921 Retrieved from the Internet: URL:https://opg.optica.org/DirectPDFAccess /0E0F1D68-3064-4F66-B5AFA9D8A2319C75_43282 4/boe-11-7-3913.pdf?da=1&id=432824&seq=0&m obile=no [retrieved on 2024-10-15] * page 1 - page 4 * * page 8 * * figures 1-4,8 * ----- | 1-22 | INV. A61B5/021 A61B5/024 A61B5/02 |
| A | US 2018/192898 A1 (ZHANG JIN [US] ET AL) 12 July 2018 (2018-07-12) * figure 5 * ----- | 1-22 | **TECHNICAL FIELDS SEARCHED (IPC)** A61B |
| A | Beeckman Simeon ET AL: "InSiDe: A Cardiovascular Screening Device Based on Silicon Photonics", SPIE Photonics Europe, 6 April 2022 (2022-04-06), XP093215861, Retrieved from the Internet: URL:https://usercontent.one/wp/www.inside- h2020.eu/wp-content/uploads/2022/05/SPIE20 22_InSiDe.pdf [retrieved on 2024-10-17] * page 30 * ----- | 1-22 | |
| A | US 11 045 271 B1 (TRAN BAO Q [US]) 29 June 2021 (2021-06-29) * claim 15 * ----- | 1-22 | |

The present search report has been drawn up for all claims

| Place of search | Date of completion of the search | Examiner |
|---|---|---|
| The Hague | 22 October 2024 | Costa Angeli, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
................................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 30 6034

This annex lists the patent family members relating to the patent documents cited in the above-mentioned European search report.
The members are as contained in the European Patent Office EDP file on
The European Patent Office is in no way liable for these particulars which are merely given for the purpose of information.

22-10-2024

| Patent document cited in search report | Publication date | Patent family member(s) | Publication date |
|---|---|---|---|
| US 2018192898 A1 | 12-07-2018 | NONE | |
| US 11045271 B1 | 29-06-2021 | NONE | |

EPO FORM P0459